# EUROPEAN PATENT SPECIFICATION

(11) **EP 2 131 784 B1**
(45) Date of publication and mention of the grant of the patent: **27.01.2016**
(21) Application number: 08717273.0
(22) Date of filing: 29.02.2008
(51) Int. Cl.: A61C 17/08

(54) **ASPIRATOR**
ASPIRATOR
ASPIRATEUR

(30) Priority: 16.03.2007 SE 0700673
(43) Date of publication of application: 16.12.2009
(73) Proprietor: Orsing, Ernst, 250 16 Råå (SE)
(72) Inventor: Orsing, Ernst, 250 16 Råå (SE)
(74) Representative: Ström & Gulliksson AB
(86) International application number: PCT/EP2008/052492
(87) International publication number: WO 2008/113670

(56) References cited:
- WO-A-2006/105973
- US-A- 2 529 499
- US-A- 3 460 255
- US-A1- 2006 199 147

## Description

### Field of the Invention

This invention pertains in general to the field of aspirators, such as saliva ejectors, suitable for ejection of saliva and particulate material in dentistry. More particularly the invention relates to an aspirator, such as a saliva ejector, and a method of manufacturing of such an aspirator, comprising a tube with an open first end to be connected with a suction device, and a second end having through inlet slots, wherein said inlet slots are formed in the wall of the tube, and that ribs formed by the wall between adjacent inlet slots are interconnected by a ring at said second end formed by the tube.

### Background of the Invention

During dental treatment a saliva ejector is usually used to suck saliva, blood and particulate material arising from dental procedures. A saliva ejector is of course also used to discard other fluids used to rinse the oral cavity in connection with said treatments.

The most commonly used saliva ejector includes a disposable barrel mounted on the suction tube and forming vacuum releasing slots to prevent the soft parts of the oral cavity from blocking the saliva ejector.

The vacuum releasing effect prevents soft tissue of the oral cavity from blocking the saliva ejector at the slots. However, the soft tissue of the oral cavity has an enormous ability to mould itself, and it is not impossible that all slots may be blocked by soft tissue. Furthermore, said assembly is accompanied by other characteristics that retard the work of the dentist. Since the slots of the barrel in the saliva ejector according to the prior art are narrow, the barrel has to be demounted from the suction tube every time particulate material needs to be discarded from the oral cavity. This is a cumbersome and seemingly unnecessary manoeuvre for the dentist or the assisting nurse. Thus, the barrel of the prior art saliva ejector needs to be removable, which presents another adverse side effect of the prior art saliva ejector, namely the risk of dropping the barrel in the oral cavity, which may lead to severe complications if the barrel enters the respiratory system.

A problem conferred to the manufacturing of aspirators, such as saliva ejectors, is that the skilled artisan wants to keep the effective suction area as small as possible, while still providing a sucking action in connection to the oral cavity isolated from the soft tissue in the oral cavity. If the suction area becomes too large the sucking action becomes inferior, and if the sucking action not is isolated from the soft tissue in the oral cavity the aspirator gets clogged.

SE 528,535 discloses an aspirator comprising a tube with an open first end to be connected with a suction device, and a second end having through inlet slots, wherein said inlet slots are formed in the wall of the tube, and that ribs formed by the wall between adjacent inlet slots are interconnected by a ring at said second end formed by the tube. However, the inlet slots, even though directed axially with regard to the lumen of the aspirator, thereby extending the suction area away from the soft tissue of the patient, may only provide sucking action as far away from the soft tissue in the oral cavity as the inlet slots extend axially from said second end.

Furthermore, the saliva ejectors according to the prior art have been manufactured in two pieces, a barrel or mouthpiece and a suction tube, since it was believed that it was impossible to manufacture these saliva ejectors in one piece, including vacuum releasing slots, by injection molding. This opinion was based on the belief that it would be impossible to eject the injection molded saliva ejector from the mold, due to too strong adherence between the mold and the molded piece.

US 3,460,255 A discloses an aspirator comprising a tube having inlet slots that differ in axial extension. US 2006/199147 A1 discloses a method of manufacturing an aspirator.

### Summary of the Invention

Accordingly, the present invention seeks to mitigate, alleviate or eliminate one or more of the above-identified deficiencies and to provide an improved aspirator of the kind referred to. For this purpose the aspirator of this kind according to claim 1, is characterized in that at least two of said inlet slots differs in length and axial extension from the second end.

Advantageous features of the invention are defined in the dependent claims.

### Brief Description of the Drawings

These and other aspects, features and advantages of which the invention is capable of will be apparent from the following description of illustrative embodiments of the present invention, reference being made to the accompanying drawings, in which
Fig. 1 is a perspective view of an aspirator according to one embodiment of the present invention,
Fig. 2a is a cross section of a second end of an aspirator according to one embodiment of the present invention,
Fig. 2b is a perspective view of a second end of an aspirator according to a second embodiment of the present invention, and
Fig. 3 is a front view of an aspirator according to the present invention.

### Description of embodiments

The following description focuses on embodiments of the present invention applicable to an aspirator, such as a saliva ejector. However, it will be appreciated that the invention is not limited to this application but may be applied to many other technical fields including for example surgery procedures, manufacturing procedures of electronics, vacuum cleaning, devices adapted for blood suction in surgical procedures etc.

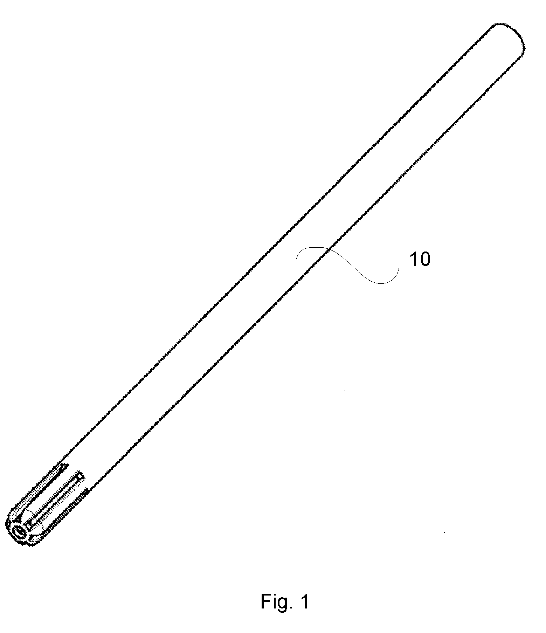

According to Fig. 1, an aspirator is provided comprising a suction tube **10** with an open first end to be connected with a suction device and a second end having six through inlet slots **11a** and **11b**, extending axially along the length of the tube **10**. The inlet slots **11a** and **11b** are formed in the wall of the tube.

The inlet slots **11a** and **11b** present the aspirator according to the invention with vacuum release characteristic in that at least one slot probably is in connection with the central passage, or lumen, of the tube. Also, slots 11a and 11b discard saliva etc. from the oral cavity of the patient during use.

Adjacent to the inlet slots **11a** and **11b** six ribs **12** are formed by the wall of the suction tube. These ribs **12** are connected by a ring **13**.

In other embodiments of the present invention the number of inlet slots **11a** and **11b** and ribs **12** may vary accordingly.

The opening of the ring **13** in the embodiments of the present invention provides the embodiments of the present invention with the possibility to evacuate particulate material from the oral cavity, without detaching the barrel from the suction tube while still presenting a vacuum releasing property.

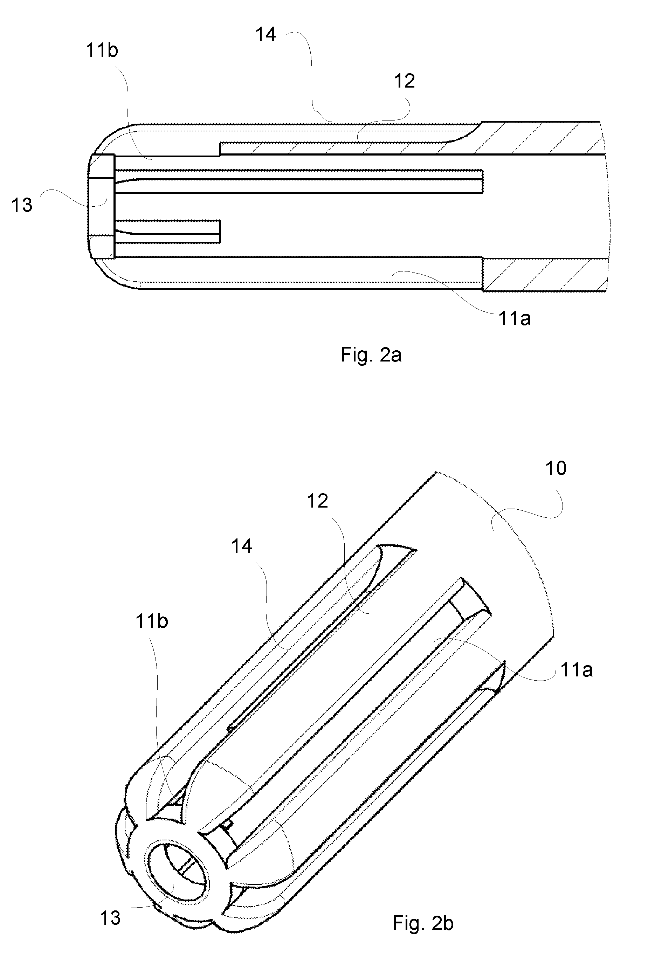

As shown in Figs. 2a and 2b the inlet slots, comprising first inlet slots **11a** and second inlet slots **11b**, extends axially from the opening of the aspirator, such as axially from the ring **13**. Thus, at least one first inlet slot **11a** and at least one second inlet slot **11b** differs in axial extension from the second end of the suction tube **10**. The axial extension of the first inlet slots **11a** is greater than the axial extension of the second inlet slots **11b** in the direction towards the first end of the suction tube **10**. In this way sucking action may be accomplished as far away from the soft tissue in the oral cavity as the first inlet slots **11a** extend. Every other inlet slot is a first inlet slot **11a** and the others are second inlet slots **11b**. In this way the aspirator may provide sucking action in the extension of the first inlet slots **11a** evenly distributed around the circumference of the aspirator. Thus, the sucking action may be provided in a greater distance from said second end of the suction tube **10**, while still providing a combined suction area allowing for satisfactory suction action.

To increase the vacuum releasing action of the second inlet slots **11b**, tracks **14** are provided in the axial extension of the second inlet slots **11b**, which is illustrated in Figs. 2a and 2b. The extension of tracks **14** may be the same as the extension of the first inlet slots **11a**. The tracks **14** provide an aid in respect of vacuum release for the second inlet slots **11b**, in the same axial extension as the first inlet slots **11a**. These tracks **14** improve the accessibility of the surrounding air to the second inlet slots **11b**, whereby the vacuum releasing ability of the second inlet slots **11b** is improved.

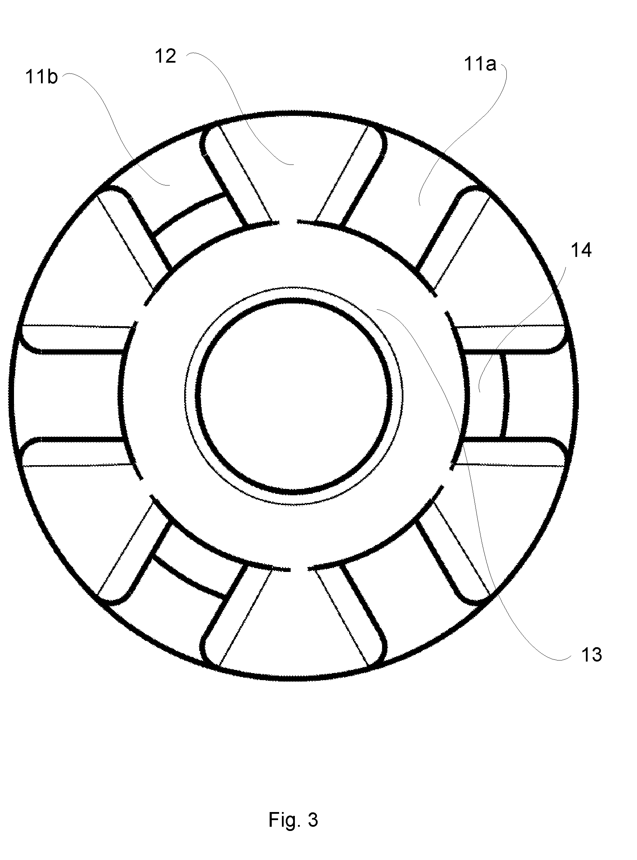

Fig. 3 illustrates a front view of the aspirator according to the present invention, wherein it is disclosed how the lumen of the aspirator extends through the ring **13**, and how the six inlet slots **11a** and **11b**, and thereby also the tracks **14**, are arranged alternatingly between the ribs **12**.

In another embodiment of the present invention the opening of the ring **13** has a diameter or size that is similar to the diameter or size of the central channel or lumen in the suction tube. This feature provides this embodiment of the present invention with the possibility to evacuate particulate material, such evacuation being limited only by the size of the lumen of the suction tube.

In still another embodiment the aspirator of the present invention is bent into a suitable form, to allow the aspirator to be placed in a sucking position in the mouth hanging on the lower jaw. This embodiment presents the advantage of relieving the dentist or the assisting nurse of holding the aspirator in a saliva sucking position during dentistry.

It has now surprisingly been discovered that the aspirator according to the present invention may be manufactured in one piece by injection moulding of a suitable material contrary to the learnings according to the prior art.

The aspirator tube according to the embodiments of the present invention is injection molded as a unitary body in a molding tool forming the aspirator tube **10** and including a mold insert forming the lumen of the tube, and a die forming the inlet slots, the mold insert and the die being mutually engaged to form the ring **13** interconnecting the ribs **12** between adjacent inlet slots **11a** and **11b**.

The die according to above is supplied with ridges in the end of the die. Some of these ridges are of a height corresponding to thickness of the wall of the tube **10**, thus being responsible for the forming of inlet slots **11a** and **11b**, and have an axial extension corresponding to the axial extension of inlet slots **11a** and **11b**, respectively, and some of these ridges, i.e. those continuing from the ridges responsible for forming inlet slots **11b**, are of a height corresponding to the depth of tracks **14**. This results in that the ridges will be located adjacent to the outer surface of the mold insert during said injection molding. Thereby, the aspirator according to the present invention is formed, and said aspirator will easily be ejected from the die.

It is also possible to use a die and/or a mold insert with slightly angled walls. This embodiment presents an aspirator tube with decreasing circumference in the direction towards the end having the through inlet slots. This aspirator will be even more easy to eject from the die.

In yet another embodiment of the present invention the aspirator tube is injection molded of a thermoplastic.

The skilled artisan is capable of determining what kind of materials that are suitable for injection moulding of the aspirator. Nevertheless, some examples of such materials may be mentioned: poly vinyl chloride (PVC), epoxy, polyvinylacetates, polylacticacids, polyhydroxyalkanoates, polyesters, polycaprolactone, polyvinylalcohol, polypropylene, polycarbonates, polyurethanes, polyethers, polyamides, polyacrylonitriles, polystyrenes, protein based plastics, and other biocompatible polymers.

Although the present invention has been described above with reference to specific illustrative embodiments, it is not intended to be limited to the specific form set forth herein. Other embodiments are possible within the scope of the appended claims.

## Claims

1. Aspirator, such as a saliva ejector, comprising a tube (10) with an open first end to be connected with a suction device, and a second end having through inlet slots (11), wherein the inlet slots (11a, 11b) are formed in the wall of the tube (10) and extending axially along said tube (10), and that ribs (12) formed by the wall between adjacent inlet slots (11a, 11b) are interconnected by a ring (13) at said second end formed by the tube (10), **cha racterized** in that said inlet slots (11a, 11b) comprises at least one first inlet slot (11a) and at least one second inlet slot (11b) that differs in length and axial extension from the second end.

2. The aspirator according to claim 1, comprising at least two first inlet slots (11a) and at least two second inlet slots (11b), wherein every other inlet slot is a first inlet slot (11a) when there is an even number of inlet slots (11a, 11b).

3. The aspirator according to claim 1 or 2, further comprising a track (14) on the outside of the tube (10), extending axially from said at least one or two second inlet slots (11b).

4. The aspirator according to any of claims 1 to 3, wherein the opening of the ring (13) is as large as the lumen of the suction tube (10).

5. The aspirator according to any of claims 1 to 4, which is made by injection moulding.

6. A method of manufacturing the aspirator according any of claims 1 to 5,
**characterised in that**
the aspirator tube is injection molded as a unitary body in a molding tool forming the aspirator tube (10) and including a mold insert forming the lumen of the tube (10), and a die forming the inlet slots (11a, 11b), the mold insert and the die being mutually engaged to form the ring (13) interconnecting the ribs (12) between adjacent inlet slots (11a, 11b).

## Patentansprüche

1. Absauger, wie beispielsweise ein Speichelabsauger, der umfasst: eine Röhre (10) mit einem offenen ersten Ende, das mit einer Saugvorrichtung verbindbar ist, und einem zweiten Ende mit durchgehenden Einlassschlitzen (11), wobei die Einlassschlitze (11a, 11b) in der Wand der Röhre (10) ausgebildet sind und sich axial entlang der Röhre (10) erstrecken, und wobei von der Wand zwischen benachbarten Einlassschlitzen (11a, 11b) ausgebildete Rippen (12) über einen von der Röhre (10) an dem zweiten Ende ausgebildeten Ring (13) miteinander verbunden sind, **dadurch gekennzeichnet, dass** die Einlassschlitze (11a, 11 b) wenigstens einen ersten Einlassschlitz (11a) und wenigstens einen zweiten Einlassschlitz (11b) umfassen, die sich in Länge und axialer Erstreckung von dem zweiten Ende unterscheiden.

2. Absauger nach Anspruch 1, der wenigstens zwei erste Einlassschlitze (11a) und wenigstens zwei zweite Einlassschlitze (11 b) umfasst, wobei jeder übernächste Einlassschlitz ein erster Einlassschlitz (11a) ist, wenn die Anzahl der Einlassschlitze (11a, 11b) gerade ist.

3. Absauger nach Anspruch 1 oder 2, der ferner eine Führung (14) an der Außenseite der Röhre (10) umfasst, die sich axial von dem wenigstens einen oder den wenigstens zwei zweiten Einlassschlitzen (11b) erstreckt.

4. Absauger nach einem der Ansprüche 1 bis 3, wobei die Öffnung des Rings (13) die Größe des Lumens der Saugröhre (10) aufweist.

5. Absauger nach einem der Ansprüche 1 bis 4, der durch Spritzgießen hergestellt ist.

6. Verfahren zum Herstellen des Absaugers nach einem der Ansprüche 1 bis 5, **dadurch gekennzeichnet, dass**
die Absaugröhre als ein einstückiger Körper in einem Formwerkzeug spritzgegossen wird, das die Absaugröhre (10) ausbildet und umfasst: einen das Lumen der Röhre (10) ausbildenden Formeinsatz und eine die Einlassschlitze (11a, 11b) ausbildende Gussform, wobei der Formeinsatz und die Gussform aneinander angreifen, um den Ring (13) auszubilden, der die Rippen (12) zwischen benachbarten Einlassschlitzen (11a, 11 b) miteinander verbindet.

## Revendications

1. Aspirateur, tel qu'une pompe à salive, comprenant un tube (10) doté d'une première extrémité ouverte à raccorder à un dispositif d'aspiration, et d'une seconde extrémité comportant des fentes d'admission traversantes (11), dans lequel les fentes d'admission (11a, 11b) sont formées dans la paroi du tube (10) et s'étendent axialement le long dudit tube (10), et des nervures (12) formées par la paroi entre les fentes d'admission adjacentes (11a, 11b) sont reliées mutuellement par un anneau (13) au niveau de ladite seconde extrémité formée par le tube (10), **caractérisé en ce que** lesdites fentes d'admission (11a, 11b) comprennent au moins une première fente d'admission (11a) et au moins une seconde fente d'admission (11b) qui diffère du point de vue de l'étendue longitudinale et axiale de la seconde extrémité.

2. Aspirateur selon la revendication 1, comprenant au moins deux premières fentes d'admission (11a) et au moins deux secondes fentes d'admission (11b), dans lequel toute autre fente d'admission est une première fente d'admission (11a) en présence d'un nombre pair de fentes d'admission (11a, 11b).

3. Aspirateur selon la revendication 1 ou 2, comprenant en outre une piste (14) sur l'extérieur du tube (10), s'étendant axialement à partir de ladite au moins une ou desdites deux secondes fentes d'admission (11).

4. Aspirateur selon l'une quelconque des revendications 1 à 3, dans lequel l'ouverture de l'anneau (13) est plus grande que la lumière du tube d'aspiration (10).

5. Aspirateur selon l'une quelconque des revendications 1 à 4, lequel est fabriqué par moulage par injection.

6. Procédé de fabrication de l'aspirateur selon l'une quelconque des revendications 1 à 5,
**caractérisé en ce que**
le tube d'aspirateur est moulé par injection en tant que corps unitaire dans un outil de moulage formant le tube (10) d'aspirateur et comprenant un insert de moule formant la lumière du tube (10), et une matrice formant les fentes d'admission (11a, 11b), l'insert de moule et la matrice coopérant mutuellement pour former l'anneau (13) reliant mutuellement les nervures (12) entre les fentes d'admission adjacentes (11a, 11b).
